# EUROPÄISCHE PATENTSCHRIFT

(11) **EP 1 368 048 B1**
(45) Veröffentlichungstag und Bekanntmachung des Hinweises auf die Patenterteilung: **14.11.2007**
(21) Anmeldenummer: 02710740.8
(22) Anmeldetag: 15.02.2002
(51) Int. Cl.: A61K 36/185, A61K 31/35, A61K 31/05, A61P 1/00, A61P 21/00, A61P 25/00

(54) **CANNABIS EXTRAKTEN PHARMAZEUTISCHE ZUSAMMENSETZUNG**
PHARMACEUTICAL COMPOSITION MADE OF CANNABIS EXTRACTS
COMPOSITION PHARMACEUTIQUE A BASE D'EXTRAITS DE CANNABIS

(30) Priorität: 06.03.2001 CH 416012001
(43) Veröffentlichungstag der Anmeldung: 10.12.2003
(73) Patentinhaber: Forschungsinstitut Hiscia Verein Für Krebsforschung, 4144 Arlesheim (CH)
(72) Erfinder: WERNER, Michael, CH-4143 Dornach (CH); SCHALLER, Gerhard, CH-4143 Dornach (CH); JÄGGY, Christoph, CH-4105 Biel-Benken (CH)
(74) Vertreter: Braun, André jr.
(86) Internationale Anmeldenummer: PCT/CH2002/000091
(87) Internationale Veröffentlichungsnummer: WO 2002/069993

(56) Entgegenhaltungen:
- ELIZABETH M. WILLIAMSON ET AL.: "CANNABINOIDS IN CLINICAL PRACTICE" DRUGS., Bd. 60, Nr. 6, Dezember 2000 (2000-12), Seiten 1303-1314, XP001024502 ADIS INTERNATIONAL LTD., AT ISSN: 0012-6667
- R. W. GORTER: "CANCER CACHEXIA AND CANNBINOIDS" FORSCHENDE KOMPLEMENTARMEDIZIN, Bd. 6, Nr. SUPPL3, Oktober 1999 (1999-10), Seiten 21-22, XP001024552 KARGER, BASEL,, CH ISSN: 1021-7096
- DATABASE BIOSIS [Online] BIOSCIENCES INFORMATION SERVICE, PHILADELPHIA, PA, US; 1989 SHOOK J E ET AL: "PSYCHOACTIVE CANNABINOIDS REDUCE GASTROINTESTINAL PROPULSION AND MOTILITY IN RODENTS" Database accession no. PREV198988028880 XP002179756 & JOURNAL OF PHARMACOLOGY AND EXPERIMENTAL THERAPEUTICS, Bd. 249, Nr. 2, 1989, Seiten 444-449, ISSN: 0022-3565
- SRIVASTAVA M D ET AL: "DELTA 9 TETRAHYDROCANNABINOL AND CANNABIDIOL ALTER CYTOKINE PRODUCTION BY HUMAN IMMUNE CELLS" IMMUNOPHARMACOLOGY, ELSEVIER SCIENCE PUBLISHERS BV, XX, Bd. 40, Nr. 3, Oktober 1998 (1998-10), Seiten 179-185, XP000957596 ISSN: 0162-3109
- MECHOULAM R ET AL: "THE CANNABINOIDS: AN OVERVIEW. THERAPEUTIC IMPLICATIONS IN VOMITINGAND NAUSEA AFTER CANCER CHEMOTHERAPY, IN APPETITE PROMOTION, IN MULTIPLE SCLEROSIS AND IN NEUROPROTECTION" PAIN RESEARCH AND MANAGEMENT, PULSUS GROUP FOR THE CANADIAN PAIN SOCIETY, OAKVILLE,, US, Bd. 6, Nr. 2, 21. Juni 2001 (2001-06-21), Seiten 67-73, XP001024550 ISSN: 1203-6765
- KALANT H: "MEDICINAL USE OF CANNABIS: HISTORY AND CURRENT STATUS UTILISATION MEDICALE DU CANNABIS: HISTORIQUE ET SITUATION ACTUELLE" PAIN RESEARCH AND MANAGEMENT, PULSUS GROUP FOR THE CANADIAN PAIN SOCIETY, OAKVILLE,, US, Bd. 6, Nr. 2, 21. Juni 2001 (2001-06-21), Seiten 80-91, XP001024544 ISSN: 1203-6765
- KORTE F.; SIEPER H.: '[ON THE CHEMICAL CLASSIFICATION OF PLANTS. XXIV. INVESTIGATION OF HASHISH CONSTITUENTS BY THIN-LAYER CHROMATOGRAPHY.]' JOURNAL OF CHROMATOGRAPHY Bd. 13, 1964, ELSEVIER PUBLISHING COMPANY, AMSTERDAM, NL, Seiten 90 - 98, XP007900328
- FRISCKNECHT H.-R. ET AL: 'Behavioral effects of Hashish in mice: II. Nursing Behavior and Development of the Sucklings' PSYCHOPHARMACOLOGY Bd. 70, Nr. 2, 1980, Seiten 155 - 161, XP009064638

## Beschreibung

Die vorliegende Erfindung betrifft eine pharmazeutische Zusammensetzung, welche ausgewählte Cannabisverbindungen der Pflanze Cannabis sativa (herba et flos sicc.) enthält. Insbesondere enthält die erfindungsgemässe Zusammensetzung die Komponenten Tetrahydrocannabinol (THC) sowie Cannabidiol (CBD) in ausgewählten Gewichtsverhältnissen, wie dies im weiteren beschrieben ist. Solche Zusammensetzungen zeigen eine überraschend hohe pharmakologische Wirkung in der palliativen Krebstherapie und bei der Behandlung von Spasmen und schmerzhaften Muskelverspannungen von Multiple-Sklerose-Patienten. Im weiteren zeigen solche Zusammensetzungen eine ausgezeichnete Schmerz lindernde Wirkung.

Die Komponenten der Cannabispflanze Cannabis sativa sind weitgehend bekannt und werden auch therapeutisch eingesetzt. So entspricht beispielsweise Δ⁹-Tetrahydrocannabinol (Δ⁹-THC) der Formel (a), und Cannabidiol (CBD) der Formel (b).

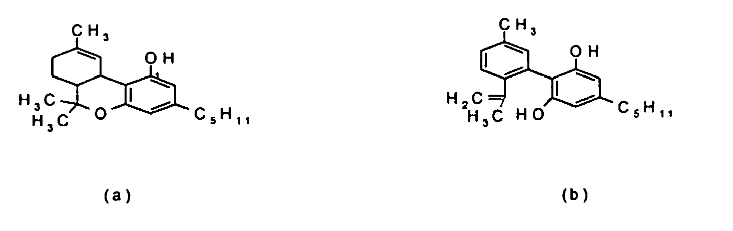

Es wurde nun gefunden, dass eine Zusammensetzung, welche mindestens 80 Gew.-%, und vorzugsweise mindestens 90 Gew.-%, THC und CBD enthält, berechnet auf das Gesamtgewicht der in der Zusammensetzung anwesenden Cannabinoide, und das Gewichtsverhältnis von THC : CBD = 75:25 bis 20:80, vorzugsweise 3:1 bis 1:2, und insbesondere 2:1 beträgt, eine überraschend hohe pharmakologische Wirkung aufweist. Dabei entsprechen die angegebenen Werte für das Gesamtgewicht der in der Zusammensetzung anwesenden Cannabinoide und die Werte für die Gewichtsverhältnisse von THC : CBD diejenigen Werten, welche über die Peakflächen aus den entsprechenden HPLC-Chromatogrammen berechnet bzw. erhalten werden.

Die vorliegende Erfindung ist in den Patentansprüchen formuliert. Insbesondere betrifft die vorliegende Erfindung eine pharmakologisch wirksame Zusammensetzung, welche für die Verwendung in der palliativen Krebstherapie und als Mittel mit muskelentspannender und/oder schmerzlindernder Wirkung bei neurologischen Erkrankungen geeignet ist, wobei die Zusammensetzung dadurch gekennzeichnet ist, dass diese mindestens 80 Gew.-%, und vorzugsweise mindestens 90 Gew.-%, Tetrahydrocannabinol (THC) und Cannabidiol (CBD) enthält, berechnet auf das Gesamtgewicht der in der Zusammensetzung anwesenden Cannabinoide, und das Gewichtsverhältnis von THC : CBD = 75:25 bis 20:80, vorzugsweise 3:1 bis 1:2, und insbesondere 2:1 beträgt.

Die vorliegende Erfindung betrifft im weiteren eine pharmakologisch wirksame Lösung oder Suspension der Wirkstoffe THC und CBD in einem geeignetem Lösungsmittel oder Suspensionsträger, wobei diese Lösung oder diese Suspension dadurch gekennzeichnet ist, dass das Gesamtgewicht von THC und CBD in dieser Lösung oder dieser Suspension 1 Gew.-% bis 25 Gew.-%, vorzugsweise 1,5 Gew.-% bis 6 Gew.-%, berechnet auf das Gesamtgewicht der Lösung oder der Suspension, beträgt, wobei der Gehalt an THC und CBD, berechnet auf das Gesamtgewicht der in der Lösung oder Suspension vorhandenen Cannabinoide, mindestens 80 Gew.-%, und vorzugsweise mindestens 90 Gew.-%, und das Gewichtsverhältnis von THC : CBD = 75:25 bis 20:80, vorzugsweise 3:1 bis 1:2, und insbesondere etwa 2:1, beträgt.

Die Erfindung betrifft im weiteren einzeldosierte Verabreichungsformen für die perorale Applikation, beispielsweise gepresste Formen, wie Tabletten oder Dragees, sowie Hartgelatinekapseln oder Weichgelatinekapseln, vorzugsweise Weichgelatinekapseln, welche die erfindungsgemässe pharmakologisch wirksame Zusammensetzung enthalten.

Die vorliegende Erfindung betrifft die erfindungsgemässe pharmakologisch wirksame Zusammensetzung in Form eines Pflanzenextraktes.

Die Erfindung betrifft im weiteren ein Verfahren zur Herstellung der erfindungsgemässen Zusammensetzung.

Die Erfindung betrifft im weiteren die Verwendung der erfindungsgemässen Zusammensetzung zur Herstellung von pharmakologisch wirksamen Mitteln zur Verwendung in der palliativen Krebstherapie und in der Behandlung MS-bedingter Spasmen.

Die vorliegende Erfindung betrifft auch die Verabreichung der erfindungsgemässen Zusammensetzung an Patienten in der palliativen Krebstherapie und als Mittel mit muskelentspannender und/oder schmerzlindernder Wirkung bei neurologischen Erkrankungen wie z.B. Multiple Sklerose.

Die erfindungsgemässe Zusammensetzung beinhaltet sowohl die auf synthetischem Wege hergestellten als auch die aus pflanzlichen Extrakten gewonnenen Verbindungen. Die Verbindung Tetrahydrocannabinol (THC) kann man beispielsweise auf synthetischem Wege herstellen. Die Synthese von THC sowie die Kontrolle der Nebenprodukte sind aber schwierig und die Reinheit des erhaltenen Produktes ist nicht optimal. Vorzugsweise werden die erfindungsgemäss verwendeten Verbindungen THC und CBD aus der Pflanze Cannabis sativa (getrocknete Blütenstände und Kraut, herba et flos sicc.) extrahiert. Gearbeitet wird jeweils immer unter Ausschluss von Sauerstoff. Dabei extrahiert man die Cannabisverbindungen aus der Pflanze in an sich bekannter Weise, beispielsweise mit niedermolekularen Alkoholen, wie Methanol, Ethanol, Butanol oder Propanol; Essigsäureestern, wie dem Methylester oder Ethylester; Ketonen, beispielsweise Aceton; Ethern, wie Methylether oder Ethylether; oder mit niedrig siedenden aliphatischen oder aromatischen oder chlorierten Kohlenwasserstoffen. Man behandelt beispielsweise die gereinigten, getrockneten und geschnittenen Pflanzen (Blütenstände, Blätter, Stengel etc) üblicherweise bei Rückflusstemperatur mit etwa der drei- bis zehnfachen Gewichtsmenge des angegebenen Lösungsmittels oder einem Gemisch solcher Lösungsmittel, vorzugsweise während mindestens etwa einer Stunde, worauf vom Rückstand abfiltriert wird. Aus dem Rückstand wird die noch vorhandene Flüssigkeit sorgfältig ausgepresst und dem Filtrat zugegeben. Das Lösungsmittel wird anschliessend, vorzugsweise unter Vakuum, beispielsweise bei einem Druck von etwa 80 mbar und bei einer Temperatur von etwa 40-60°C, entfernt. Das erhaltene Extrakt, welches mit diesem Verfahren in der Regel als honigartiges Harz anfällt, wird nun während etwa 40 Minuten auf eine Temperatur von etwa 110°C bis etwa 135°C, vorzugsweise bei etwa 120°C, vorzugsweise in einem Autoklaven, erhitzt. Bei dieser Temperatur werden die Verbindungen, welche im Extrakt als Carbonsäuren vorliegen, decarboxyliert, wobei die Verbindungen THC und CBD in praktisch quantitative Ausbeute gebildet werden. Anschliessend wird das abgekühlte Produkt vorzugsweise in Petrolether aufgenommen und einer Chromatographie an Kieselgel mit einem geeigneten Laufmittel, beispielsweise mit einer Petrolether/Ethylacetat-Mischung, unterzogen. Die erhaltene Cannabinoidfraktion, die mit Hilfe der Dünnschichtchromatographie detektiert werden kann, wird anschliessend einer chromatographischen Trennung an einem hydrophobierten Kieselgel, beispielsweise an octadecylsilyliertem Kieselgel, unterzogen, wobei zur Chromatographie vorzugsweise ein Laufmittelgemisch bestehend aus Methanol/Wasser und Essigsäure oder Ethanol/Wasser und Essigsäure, verwendet wird. Mit dieser Reinigung erhält man gereinigtes CBD als eine erste Fraktion und gereinigtes THC als zweite Fraktion. Die Reinheit der erhaltenen Verbindungen bzw. Wirkstoffe wird mit Hilfe von HPLC bestimmt, wobei in der Regel eine Reinheit von mindestens 90 Gew.-% bezogen auf das Gesamtgewicht der in dieser Fraktion anwesenden Komponenten erhalten wird.

Man kann alternativ aber auch so vorgehen, dass man die Cannabisverbindungen aus den gereinigten, getrockneten und geschnittenen Pflanzenteilen in an sich bekannter Weise mit etwa der drei- bis zehnfachen Gewichtsmenge eines organischen Lösungsmittels, welches in Wasser nicht löslich ist, das heisst mit Wasser ein Zweiphasensystem bildet, extrahiert. Gearbeitet wird auch hier jeweils unter Ausschluss von Sauerstoff. Geeignete Lösungsmittel sind beispielsweise wasserunlösliche Essigsäureester, vorzugsweise die Methyl- oder Ethylester von Essigsäure; wasserunlösliche Ether, wie beispielsweise Diethylether oder Ethylpropylether; oder aliphatische oder aromatische oder chlorierte Kohlenwasserstoffe. Das organische Lösungsmittel, welches die extrahierten Cannabisverbindungen enthält, wird nun filtriert und anschliessend mindestens zweimal mit einer 2 %-igen wässrigen Natriumhydroxidlösung, welche vorzugsweise etwa 20 Gew.-% Ethanol enthält, extrahiert. Dabei gehen insbesondere diejenigen Cannabisverbindungen in die wässerig/alkoholische Phase über, welche eine Carboxylgruppe enthalten. Die vereinigten wässrig/ethanolischen Phasen werden nun mit einer 5 %igen Schwefelsäurelösung versetzt, so dass ein Säurewert (pH-Wert) von etwa 2-4 entsteht, worauf mit einem niedrig siedenden lipophilen Lösungsmittel, beispielsweise mit einem niedrig siedenden aliphatischen oder aromatischen oder chlorierten Kohlenwasserstoff, einem Essigsäureester, wie z.B. dem Methyl- oder Ethylester von Essigsäure oder mit einem Ether, oder mit einem Gemisch solcher Verbindungen, mindestens zweimal extrahiert wird. Das Lösungsmittel wird nun im Vakuum bei niedriger Temperatur entfernt. Anschliessend wird der Rückstand einer chromatographischen Trennung an einem hydrophobierten Kieselgel, beispielsweise an octadecylsilyliertem Kieselgel, unterzogen, wobei zur Chromatographie vorzugsweise ein Laufmittelgemisch bestehend aus Methanol/Wasser und Essigsäure oder Ethanol/Wasser und Essigsäure, verwendet wird. Detektiert wird mit einem UV-Detektor bei 280 nm. Es werden die wirkstoffhaltigen Fraktionen mit den nativen Substanzen CBD-Säure und THC-Säure erhalten. Die Identifikation und die Bestimmung der Reinheit der Fraktionen erfolgt mit Hilfe von HPLC. Das in den Fraktionen enthaltene Extraktionsmittel (Lösungsmittel) wird nun unter Vakuum entfernt. Vorzugsweise werden die Wirkstoffe bzw. die nativen Ausgangssubstanzen (d.i. CBD-Säure und THC-Säure) mit einem lipophilen Lösungsmittel oder einem Suspensionsträger aufgenommen. Anschliessend werden die Wirkstoffe bzw. Wirkstofflösungen während etwa 40 Minuten unter Sauerstoffausschluss auf etwa 110°C bis etwa 135°C, vorzugsweise bei etwa 120°C, vorzugsweise in einem Autoklaven, erhitzt. Bei dieser Temperatur werden die Verbindungen, welche im Extrakt als Carbonsäuren vorliegen, decarboxyliert, wobei die Verbindungen THC und CBD in praktisch quantitative Ausbeute gebildet werden. Die Identifikation der Komponenten kann mit an sich bekannten Methoden vorgenommen werden, beispielsweise mit Dünnschicht-Chromatographie (Thin-Layer-Chromatography, TLC). Für die Bestimmung der Reinheit und des Gehaltes verwendet man mit Vorteil High Pressure Liquid Chromatography (HPLC).

Geeignete lipophile Lösungsmittel oder Suspensionsträger sind beispielsweise mittel- und/oder kurzkettige Triglyceride, mittelkettige Partialglyceride, polyoxethylierte Fettalkohole, polyoxethylierte Fettsäuren, polyoxethylierte Fettsäuretriglyceride oder Partialglyceride, Ester von Fettsäuren mit niedermolekularen Alkoholen, Partialester von Sorbitan mit Fettsäuren, polyoxethylierte Partialester von Sorbitan mit Fettsäuren, Partialester von Zuckern oder oligomeren Zuckern mit Fettsäuren, Polyethylenglykole, sowie Mischungen der genannten Verbindungen. Geeignet sind auch Mischungen der genannten Verbindungen mit Fetten, Ölen und/oder Wachsen oder Glykolen oder Suspensionen in Mischungen von Lecithinen und/oder Ölen und/oder Wachsen.

Mit dem oben beschriebenen Extraktionsverfahren erhält man in der Regel Extrakte, bzw. Extraktfraktionen, welche jeweils die Komponenten Tetrahydrocannabinol (THC) oder Cannabidiol (CBD) in einer Menge von mindestens 90 Gew.-%, berechnet auf das Gesamtgewicht der im Extrakt vorhandenen Cannabinoide, enthalten. Die verbleibenden Gewichtsanteile bestehen aus andern in der Cannabispflanze anwesenden Verbindungen.

Zur Herstellung der erfindungsgemässen Mischung geht man vorzugsweise so vor, dass man die beiden, den Wirkstoff enthaltenden, Lösungen im entsprechenden Verhältnis zusammen mischt, so dass ein Verhältnis der Wirkstoffe THC:CBD im Bereich von 75:25 bis 80:20, vorzugsweise 3:1 bis 1:2, und insbesondere etwa 2:1, erhalten wird.

Dabei liegt der gesamte Gehalt an Tetrahydrocannabinol (THC) und Cannabidiol (CBD) in der pharmakologisch wirksamen Lösung oder Suspension vorzugsweise im Bereich von 1 Gew.-% bis 25 Gew.-%, vorzugsweise im Bereich von 1 Gew.-% bis 6 Gew.-% und insbesondere im Bereich von 1,5 Gew.-% bis 6 Gew.-%, bezogen auf das Gewicht sämtlicher Bestandteile der Lösung bzw. der Suspension. Bei der Herstellung von Tabletten oder Dragees bleibt das Verhältnis der Wirkstoffe THC:CBD wie oben angegeben, deren Konzentration bezogen auf das Gesamtgewicht der Tablette oder der Dragées kann aber höher sein.

In der therapeutischen Anwendung der erfindungsgemässen Zusammensetzung werden in der Indikation palliative Krebstherapie als therapeutische Dosis täglich im Durchschnitt 5 mg THC, in einem Dosierungsspektrum von 2,5-20 mg THC verabreicht (dies entspricht 3,75-120 mg der erfindungsgemässen Zusammensetzung, berechnet auf das Trockengewicht von THC und CBD). In der Indikation MS-bedingte Spasmen werden als therapeutische Dosis täglich im Durchschnitt 10 mg THC, in einem Dosierungsspektrum von 5-30 mg THC verabreicht (dies entspricht 7,5-150 mg der erfindungsgemässen Zusammensetzung).

Die erfindungsgemässe Zusammensetzung hat zwei hauptsächliche Anwendungsgebiete, nämlich (i) die palliative Krebstherapie (Appetitlosigkeit/Gewichtsverlust, Übelkeit/Erbrechen, chronische Schmerzen und reaktive Depression) und (ii) muskelentspannende und/oder schmerzlindernde Wirkung bei neurologischen Erkrankungen, insbesondere der Multiple Sklerose.

Den pharmakologischen Wirkungen der erfindungsgemässen Zusammensetzung können weitere Anwendungsgebiete wie folgt zugeordnet werden:
- Appetitstimulierende Wirkung: Die appetitstimulierende Wirkung der erfindungsgemässen Zusammensetzung kann auch bei Anorexie/Kachexie von HIV-positiven Patienten (AIDS-Wasting) und bei der postoperativen Umstellung von Patienten (insbesondere von längere Zeit beatmeten) auf die orale Ernährung therapeutisch genutzt werden.
- Antiemetische (übelkeitshemmende) Wirkung: Die antiemetische Wirkung der erfindungsgemässen Zusammensetzung kann auch gegen Übelkeit/ Erbrechen infolge von (kurativ intendierter) Chemotherapie bei Krebspatienten (insbesondere als adjuvante Antiemesis im Rahmen einer Behandlung mit 5HT3-Antagonisten) und in der antiemetischen Supporttherapie bei HIV-Infektion/AIDS und Hepatitis B genutzt werden.
- Analgetische (schmerzlindernde) Wirkung: Die analgetische Wirkung der erfindungsgemässen Zusammensetzung kann auch bei chronischen Schmerzen anderer Ursache als einer fortgeschrittenen Krebserkrankung bzw. einer neurologischen Erkrankung, so z.B. bei Migräne, Erkrankungen des Bewegungsapparats sowie des Binde- und Muskelgewebes (Arthrose, Arthritis, Myopathien), bei Menstruationsbeschwerden, bei Magen- und Darmbeschwerden (z.B. Morbus Crohn) und bei Phantomschmerzen therapeutisch genutzt werden. Besonders hervorzuheben ist die analgetische Wirkung der erfindungsgemässen Zusammensetzung bei neuropathischen Schmerzen, insbesondere bei Zoster-Neuralgie.
- Antidepressive (stimmungsaufhellende) und anxiolytische (angstlösende) Wirkung: Die antidepressive und anxiolytische Wirkung der erfindungsgemässen Zusammensetzung kann auch zur unterstützenden Behandlung anderer chronischer oder nicht (mehr) heilbarer Erkrankungen wie AIDS, Querschnittslähmung oder chronische Polyarthritis genutzt werden.
- Andere pharmakologische Wirkungen der erfindungsgemässen Zusammensetzung sind eine beruhigende/schlaffördernde Wirkung (Schlaflosigkeit), eine antiepileptische Wirkung (Epilepsien), eine bronchienerweiternde Wirkung (Asthma bronchiale), eine Modulation der motorischen Abläufe (neurologische Bewegungsstörungen wie z.B. Dystonien, Tourette-Syndrom), eine Senkung des Augeninnendrucks (Glaukom) und eine antiinflammatorische (entzündungshemmende) Wirkung (Morbus Crohn, Colitis ulcerosa, Arthritis, Neurodermitis).

Die genannten pharmakologischen Wirkungen gehen in der Mehrzahl der Fälle primär vom THC-Anteil in der erfindungsgemässen Zusammensetzung aus, werden jedoch durch den CBD-Anteil entscheidend moduliert, modifiziert und somit in seinen positiven Auswirkungen verstärkt. CBD führt durch seine spezifisch anxiolytische, antihalluzinogene und antipsychotische Wirkung darüber hinaus zu einer deutlichen Reduzierung von Nebenwirkungen, die bei Gabe von isoliertem THC beobachtet werden können, und damit zur Verbesserung der Verträglichkeit der erfindungsgemässen Zusammensetzung.

Da die erfindungsgemässe Zusammensetzung im Betäubungsmittelgesetz als nicht verkehrsfähige Substanz geführt wird, die ausschliesslich im Rahmen von klinischen Studien eingesetzt werden darf, konnte ihre Anwendung bis zum November 2000 nur auf den beiden Hauptanwendungsgebieten (palliative Krebstherapie und Muskelspasmen bei Multipler Sklerose) realisiert werden. Eine multizentrische, randomisierte, doppelblinde, placebokontrollierte Studie zum Vergleich der erfindungsgemässen Zusammensetzung mit isoliertem THC in deren Wirkung auf Anorexie/Kachexie, Übelkeit und reaktive Depression sowie in deren Verträglichkeit bei Krebspatienten in palliativer Situation wird voraussichtlich im Herbst 2001 abgeschlossen werden, wobei positive Resultate zu erwarten sind, insbesondere was die Wirkung der erfindungsgemässen Zusammensetzung im Vergleich mit der Wirkung von THC betrifft.

Die folgenden Beispiele erläutern die Erfindung.

### Beispiel 1 (Herstellung der einzelnen THC- und CBD-Fraktionen)

a) 360 Gramm getrocknetes und gereinigtes Pflanzenmaterial der Pflanze Cannabis sativa L. (flos et herba sicc., getrocknete Blütenstände und Kraut) wurden mit 3600 Gramm Ethanol (96 %-ig) gemischt und am Rückfluss bei Normaldruck während einer Stunde extrahiert. Nach dem Abkühlen wurde das Pflanzenmaterial von der Flüssigkeit abgepresst und das Filtrat filtriert.
b) Aus der in Abschnitt a) erhaltenen Lösung wurde das Lösungsmittel unter reduziertem Druck bei 10³ Pa (=100 bar) und bei 40°C entfernt und der erhaltenen harzartige Rückstand in einem Autoklaven während 40 Minuten bei einer Temperatur von 120°C erhitzt. Nach dem Abkühlen wurde das Harz in Petrolether aufgenommen und von allfälligen Rückständen filtriert.

Durch Chromatographie an Kieselgel (0,035-0,070 mm, Chromatographie mit einem Laufmittelgemisch von 1 Gew.-% Ethylacetat in Petrolether) wurde eine Cannabinoidfraktion erhalten, die mittels DC detektiert wurde [Kieselgel 60 F254 (HPTLC-Fertigplatte), Hexan/Diethylether 8:2; Detektion: 125 mg Echtblausalz B in 30 ml 1 N Natronlauge, dest. Wasser ad 300 ml, Auswertung bei Tageslicht]. Die Isolierung der THC- und CBD-Fraktion erfolgte mittels präparativer HPLC an octadecyl-silyliertem Kieselgel (LiChrospher 100 RP18, 7 µm) mit 70 Gew.-% Ethanol und 1 Gew.-% Eisessig in Wasser als Elutionsmittel. Die Detektion erfolgte bei 280 nm. Nach dem Verdampfen des Elutionsmittels wurde eine THC-Fraktion und eine CBD-Fraktion erhalten. Die Reinheit der Fraktionen wurde mittels HPLC bestimmt. Dabei wurde für beide Fraktionen (THC und CBD) eine Reinheit von mindestens 90 Gew.-%, bezogen auf das Gewicht der erhaltenen Trockensubstanz, gemessen.

### Beispiel 2 (Herstellung einer Verabreichungsform)

Die gemäss Beispiel 1, Abschnitt b) erhaltenen Komponenten wurden im Verhältnis 2.5 mg THC und 1.25 mg CBD (jeweils berechnet als Trockensubstanz) gemischt. Die Mischung wurde mit mittelkettigen Partialglyceriden (Imwitor 742 der Hüls AG; chemische Zusammensetzung: Mono- und Diglyceride von C₈-C₁₂-Fettsäuren) aufgenommen, so dass eine Konzentration von 10 mg THC/Gramm Lösung erhalten wurde. Die derart erhaltene Lösung wurde, unter Verwendung einer an sich bekannten Abfüllapparatur für Weichgelatinekapseln, in Weichgelatinekapseln abgefüllt, wobei Weichgelatinekapseln mit einem Gehalt von 2.5 mg THC und 1.25 mg CBD pro Kapsel erhalten wurden.

## Patentansprüche

1. Pharmakologisch wirksame Zusammensetzung, welche für die Herstellung von pharmakologisch wirksamen Mitteln zur Verwendung in der palliativen Krebstherapie und als Mittel mit muskelentspannender und/oder schmerzlindernder Wirkung in der Behandlung von neurologischen Erkrankungen, insbesondere MS-bedingter Spasmen, geeignet ist, **dadurch gekennzeichnet, dass** diese Zusammensetzung mindestens 90 Gew.-%, Tetrahydrocannabinol (THC) und Cannabidiol (CBD) enthält, berechnet auf das Gesamtgewicht der in der Zusammensetzung anwesenden Cannabinoide, und das Gewichtsverhältnis von THC : CBD = 3:1 bis 1:2 beträgt.

2. Zusammensetzung nach Anspruch 1, **dadurch gekennzeichnet, dass** das Gewichtsverhältnis von THC : CBD = 2:1 beträgt.

3. Zusammensetzung nach Anspruch 1, **dadurch gekennzeichnet, dass** mindestens eine der Verbindungen THC oder CBD auf synthetischem Wege hergestellt wurde.

4. Zusammensetzung nach Anspruch 1, **dadurch gekennzeichnet, dass** die Verbindungen THC und CBD aus pflanzlichen Extrakten gewonnenen wurden.

5. Zusammensetzung nach einem der Ansprüche 1-4, **dadurch gekennzeichnet, dass** die Verbindungen THC und CBD als Pflanzenextrakt aus der Pflanze Cannabis sativa (getrocknete Blütenstände und Kraut, herba et flos sicc.) gewonnen wurden.

6. Zusammensetzung nach einem der Ansprüche 1-5, **dadurch gekennzeichnet, dass** die Verbindungen THC und CBD in einem lipophilen Lösungsmittel oder Suspensionsträger gelöst sind.

7. Zusammensetzung nach Anspruch 6, **dadurch gekennzeichnet, dass** das lipophile Lösungsmittel oder der lipophile Suspensionsträger ein mittel- und/oder kurzkettiges Triglycerid, ein mittelkettiges Partialglycerid, einen polyoxethylierten Fettalkohol, eine polyoxethylierte Fettsäure, ein polyoxethyliertes Fettsäuretriglycerid oder Partialglycerid, einen Ester von Fettsäuren mit niedermolekularen Alkoholen, einen Partialester von Sorbitan mit Fettsäuren, einen polyoxethylierte Partialester von Sorbitan mit Fettsäuren, einen Partialester von Zuckern oder oligomeren Zuckern mit Fettsäuren, ein Polyethylenglykol, sowie Mischungen dieser Verbindungen oder Mischungen dieser Verbindungen mit Fetten, Ölen und/oder Wachsen oder Glykolen oder Suspensionen in Mischungen von Lecithinen und/oder Ölen und/oder Wachsen, darstellt.

8. Verfahren zur Herstellung einer Zusammensetzung gemäss einem der Ansprüche 1-7, **dadurch gekennzeichnet, dass** man die beiden, den Wirkstoff enthaltenden, Lösungen derart mischt, dass das Gewichtsverhältnis der Wirkstoffe THC:CBD im Bereich von 3:1 bis 1:2 liegt.

9. Verfahren nach Anspruch 8, **dadurch gekennzeichnet, dass** das Gewichtsverhältnis der Wirkstoffe THC:CBD im Bereich von 2:1 liegt.

10. Pharmakologisch wirksame Lösung oder Suspension, enthaltend eine Zusammensetzung gemäss einem der Ansprüche 1-7, **dadurch gekennzeichnet, dass** deren Gesamtgehalt an Tetrahydrocannabinol (THC) und Cannabidiol (CBD) im Bereich von 1 Gew.-% bis 25 Gew.-%, vorzugsweise im Bereich von 1,5 Gew.-% bis 6 Gew.-%, bezogen auf das Gewicht sämtlicher Bestandteile, liegt.

11. Einzeldosierte Verabreichungsformen für die perorale Applikation in gepresster Form, vorzugsweise als Tablette oder Dragee, oder in Form von Hartgelatinekapseln oder Weichgelatinekapseln, vorzugsweise in Form von Weichgelatinekapseln, welche eine Zusammensetzung gemäss einem der Ansprüche 1-7 enthalten.

12. Verabreichungsform nach Anspruch 11, **dadurch gekennzeichnet, dass** diese eine Zusammensetzung gemäss den Ansprüchen 1-7 in einer Menge von etwa 3.75 mg bis etwa 35.5 mg, vorzugsweise von etwa 7.5 mg bis etwa 25 mg (berechnet auf das Trockengewicht der Summe von THC und CBD) enthalten.

13. Verwendung einer Zusammensetzung enthaltend Tetrahydrocannabinol (THC) und Cannabidiol (CBD), für die Herstellung von pharmakologisch wirksamen Mitteln zur Verwendung in der palliativen Krebstherapie und als Mittel mit muskelentspannender und/oder schmerzlindernder Wirkung in der Behandlung von neurologischen Erkrankungen, insbesondere MS-bedingter Spasmen, **dadurch gekennzeichnet, dass** diese Zusammensetzung mindestens 90 Gew.-%, Tetrahydrocannabinol (THC) und Cannabidiol (CBD) enthält, berechnet auf das Gesamtgewicht der in der Zusammensetzung anwesenden Cannabinoide, und das Gewichtsverhältnis von THC : CBD = 3:1 bis 1:2 beträgt.

14. Verwendung nach Anspruch 13, **dadurch gekennzeichnet, dass** das Gewichtsverhältnis von THC : CBD = 2:1 beträgt.

15. Verwendung einer Zusammensetzung gemäss einem der Ansprüche 2-7 für die Herstellung von pharmakologisch wirksamen Mitteln zur Verwendung nach Anspruch 13.

## Claims

1. Pharmacologically active composition which is suitable for producing pharmacologically active remedies for use in palliative cancer therapy and as remedy with a muscle-relaxing and/or pain relieving effect in the treatment of neurological disorders, in particular MS-related spasms, this composition comprises at least 90% by weight of tetrahydrocannabinol (THC) and cannabidiol (CBD), calculated from the total weight of the cannabinoids present in the composition, and the THC:CBD ratio by weight is 3:1 to 1:2.

2. Composition according to Claim 1, **characterized in that** the THC:CBD ratio by weight is 2:1.

3. Composition according to Claim 1, **characterized in that** at least one of the compounds THC or CBD has been produced by a synthetic route.

4. Composition according to Claim 1, **characterized in that** the compounds THC and CBD have been obtained from plant extracts.

5. Composition according to any of Claims 1-4, **characterized in that** the compounds THC and CBD have been obtained as plant extract from the plant Cannabis sativa (dried inflorescences and plant, herba et flos sicc.).

6. Composition according to any of Claims 1 to 5, **characterized in that** the compounds THC and CBD are dissolved in a lipophilic solvent or suspension carrier.

7. Composition according to Claim 6, **characterized in that** the lipophilic solvent or the lipophilic suspension carrier is a medium and/or short-chain triglyceride, a medium-chain partial glyceride, a polyoxyethylated fatty alcohol, a polyoxyethylated fatty acid, a polyoxyethylated fatty acid triglyceride or partial glyceride, an ester of fatty acids with low molecular weight alcohols, a partial ester of sorbitan with fatty acids, a polyoxyethylated partial ester of sorbitan with fatty acids, a partial ester of sugars or oligomeric sugars with fatty acids, a polyethylene glycol, and mixtures of these compounds or mixtures of these compounds with fats, oils and/or waxes or glycols or suspensions in mixtures of lecithins and/or oils and/or waxes.

8. Process for producing a composition according to any of Claims 1-7, **characterized in that** the two solutions containing the active ingredient are mixed in such a way that the ratio by weight of the active ingredients THC:CBD is in the range from 3:1 to 1:2.

9. Process according to Claim 8, **characterized in that** the ratio by weight of the active ingredients THC:CBD is in the range of 2:1.

10. Pharmacologically active solution or suspension comprising a composition according to any of Claims 1-7, **characterized in that** its total content of tetrahydrocannabinol (THC) and cannabidiol (CBD) is in the range from 1% by weight to 25% by weight, preferably in the range from 1.5% by weight to 6% by weight, based on the weight of all the ingredients.

11. Single-dose administration form for oral administration in compressed form, preferably as tablet or coated tablet, or in the form of hard gelatin capsules or soft gelatin capsules, preferably in the form of soft gelatin capsules, which comprise a composition according to any of Claims 1-7.

12. Administration form according to Claim 11, **characterized in that** it comprises a composition according to Claims 1-7 in an amount of about 3.75 mg to about 35.5 mg, preferably of about 7.5 mg to about 25 mg (calculated from the dry weight of the total of THC and CBD).

13. Use of a composition comprising tetrahydrocannabinol (THC) and cannabidiol (CBD), for producing pharmacologically active remedies for use in palliative cancer therapy and as remedy with a muscle-relaxing and/or pain-relieving effect in the treatment of neurological disorders, in particular MS-related spasms, **characterized in that** this composition comprises at least 90% by weight of tetrahydrocannabinol (THC) and cannabidiol (CBD), calculated from the total weight of the cannabinoids, present in the composition, and the THC:CBD ratio by weight is 3:1 to 1:2.

14. Use according to Claim 13, **characterized in that** the THC:CBD ratio by weight is 2:1.

15. Use of a composition according to any of Claims 2 to 7 for producing pharmacologically active remedies for use according to Claim 13.

## Revendications

1. Composition pharmacologiquement efficace, appropriée pour la fabrication de produits pharmacologiquement efficaces pour l'utilisation dans la thérapie palliative contre le cancer et comme produit avec un effet qui détend les muscles et/ ou qui atténue les douleurs dans le traitement de maladies neurologiques, en particulier des spasmes dus à la sclérose en plaques, **caractérisée en ce que** cette composition comprend au moins 90 % en poids de tétrahydrocannabinol (THC) et de cannabidiol (CBD), calculé par rapport au poids total de cannabinoïdes présents dans la composition et que la proportion en poids de THC : CBD = 3 : 1 à 1 : 2.

2. Composition selon la revendication 1, **caractérisée en ce que** la proportion en poids de THC: CBD = 2 : 1.

3. Composition selon la revendication 1, **caractérisée en ce qu'**au moins l'un des composés THC ou CBD a été fabriqué de manière synthétique.

4. Composition selon la revendication 1, **caractérisée en ce que** les composés THC et CBD ont été obtenus à partir d'extraits végétaux.

5. Composition selon l'une des revendications 1 à 4, **caractérisée en ce que** les composés THC et CBD ont été obtenus en tant qu'extrait végétal de la plante cannabis sativa (inflorescence séchée et herbe, herba et flos sicc.).

6. Composition selon l'une des revendications 1 à 5, **caractérisée en ce que** les composés THC et CBD sont dissouts dans un solvant ou un agent porteur de suspension lipophiles.

7. Composition selon la revendication 6, **caractérisée en ce que** le solvant lipophile ou l'agent porteur de suspension lipophile sont un triglycéride à chaînes moyennes et/ ou courtes, un glycéride partiel à chaînes moyennes, un alcool gras polyoxyéthylé, un acide gras polyoxyéthylé, un glycéride d'acide gras ou glycéride partiel polyoxyéthylés, un ester d'acides gras avec des alcools de faibles masses moléculaires, un ester partiel de sorbitan avec des acides gras, un ester partiel polyoxyéthylé de sorbitan avec des acides gras, un ester partiel de sucres ou de sucres oligomères avec des acides gras, un polyéthylèneglycol, ainsi que des mélanges de ces composés ou des mélanges de ces composés avec des graisses, des huiles et/ ou des cires ou des glycols ou des suspensions dans des mélanges de lécithines et/ ou d'huiles et/ ou de cires.

8. Procédé pour la fabrication d'une composition selon l'une quelconque des revendications 1 à 7, **caractérisé en ce que** l'on mélange les deux solutions qui contiennent la substance active de telle manière que la proportion en poids des substances actives THC : CBD est comprise entre 3 : 1 et 1 : 2.

9. Procédé selon la revendication 8, **caractérisé en ce que** la proportion en poids des substances actives THC : CBD est de l'ordre de 2 : 1.

10. Solution ou suspension pharmacologiquement efficaces, comprenant une composition selon l'une quelconque des revendications 1 à 7, **caractérisées en ce que** la teneur totale en tétrahydrocannabinol (THC) et en cannabidiol (CBD) est comprise entre 1 % en poids et 25 % en poids, de préférence comprise entre 1,5 % en poids et 6 % en poids, calculée par rapport au poids total des composants.

11. Formes d'administration en doses individuelles pour l'application pérorale dans une forme pressée, de préférence en comprimé ou en dragée, ou sous forme de capsules en gélatine dure ou de capsules en gélatine molle, de préférence sous forme de capsules en gélatine molle, qui contiennent une composition selon l'une quelconque des revendications 1 à 7.

12. Forme d'administration selon la revendication 11, **caractérisée en ce que** cette forme d'administration comprend une composition selon l'une quelconque des revendications 1 à 7 en une quantité d'environ 3.75 mg à environ 35.5 mg, de préférence d'environ 7.5 mg à environ 25 mg (calculée par rapport au poids sec de la somme de THC et CBD).

13. Utilisation d'une composition comprenant du tétrahydrocannabinol (THC) et du cannabidiol (CBD) pour la fabrication de produits pharmacologiquement efficaces pour l'utilisation dans la thérapie palliative contre le cancer et comme produit avec un effet qui détend les muscles et/ ou qui atténue les douleurs dans le traitement de maladies neurologiques, en particulier des spasmes dus à la sclérose en plaques, **caractérisée en ce que** cette composition comprend au moins 90 % en poids de tétrahydrocannabinol (THC) et de cannabidiol (CBD), calculé par rapport au poids total de cannabinoïdes présents dans la composition et que la proportion en poids de THC : CBD = 3 : 1 à 1 : 2.

14. Utilisation selon la revendication 13, **caractérisée en ce que** la proportion en poids de THC : CBD = 2 : 1.

15. Utilisation d'une composition selon l'une quelconque des revendications 2 à 7 pour la fabrication de produits pharmacologiquement efficaces pour l'utilisation selon la revendication 13.
